# EUROPÄISCHE PATENTANMELDUNG

(11) **EP 4 427 606 A1**
(43) Veröffentlichungstag der Anmeldung: **11.09.2024**
(21) Anmeldenummer: 24161430.4
(22) Anmeldetag: 05.03.2024
(51) Int. Cl.: A24F 15/00, B65D 85/10

(54) **ANORDNUNG ZUR AUFNAHME EINES ERHITZBAREN UND/ODER RAUCHBAREN MATERIALS, INSBESONDERE TABAKMATERIALS, WELCHES WENIGSTENS EIN CANNABINOID ODER WENIGSTENS EINE WENIGSTENS EIN CANNABINOID ENTHALTENDE VERBINDUNG ENTHÄLT**

(30) Priorität: 06.03.2023 DE 102023105426; 17.03.2023 DE 102023106761; 05.04.2023 DE 202023101733 U
(71) Anmelder: Biological Health Care LLC, Lewes, DE 19958 (US)
(72) Erfinder:
(74) Vertreter: Hafner & Kohl PartmbB

(57) **Zusammenfassung**

Anordnung (1) zur Aufnahme eines erhitzbaren und/oder rauchbaren Materials (2), insbesondere Tabakmaterials, welches wenigstens ein Cannabinoid oder wenigstens eine wenigstens ein Cannabinoid enthaltende Verbindung enthält.

## Beschreibung

Die Erfindung betrifft eine Anordnung zur Aufnahme eines erhitzbaren und/oder rauchbaren Materials, insbesondere Tabakmaterials, welches wenigstens ein Cannabinoid oder wenigstens eine wenigstens ein Cannabinoid enthaltende Verbindung enthält.

Im Zuge der fortschreitenden Verbreitung des Konsums von erhitzbarem und/oder rauchbarem Material, welches wenigstens ein Cannabinoid oder wenigstens eine wenigstens ein Cannabinoid enthaltende Verbindung enthält, besteht ein Bedarf nach Anordnungen zur Aufnahme entsprechender Materialien. Entsprechende Anordnungen sollen auch eine sichere Lagerung und/oder einen sicheren Transport entsprechender Materialien ermöglichen.

Die Anordnungen sollten so beschaffen sein, dass sie einem Verwender - hierbei kann es sich z. B. um eine Einrichtung in einer technischen Prozesskette, welche z. B. Herstellungs- und/oder Lieferprozesse beinhaltet, handeln - Sicherheit im Hinblick auf die Ausführung von in einer jeweiligen Prozesskette anfallenden technischen Prozessen verschafft. Die Sicherheit soll insbesondere darin bestehen, dass dem Verwender verlässliche Informationen, d. h. insbesondere technische Informationen, zu dem in der Anordnung aufzunehmenden oder aufgenommenen Material bereitgestellt werden, welche dieser im Rahmen der Ausführung von in einer jeweiligen Prozesskette anfallenden technischen Prozessen berücksichtigen kann oder muss.

Analoges gilt für weitere Gruppen von Verwendern, wie z. B. Endnutzer bzw. Konsumenten entsprechender erhitzbarer und/oder rauchbarer Materialien oder Personen, wie z. B. Ärzte, Apotheker, Händler, etc., die Endnutzern bzw. Konsumenten entsprechende erhitzbare und/oder rauchbare Materialien zur Verfügung stellen, denn auch diesen Gruppen soll Sicherheit verschafft werden, indem ihnen verlässliche Informationen, d. h. insbesondere chemische und/oder physikalische Informationen, zu dem in der Anordnung aufzunehmenden oder aufgenommenen Material bereitgestellt werden.

Das vorstehend beschriebene Anforderungsprofil ist durch bekannte Anordnungen zur Aufnahme entsprechender Materialien nicht erfüllt, als diese in der Regel einfache Dosen, Schachteln oder Tüten darstellen, die lediglich mit, bisweilen z. B. von einem Händler selbst gewählten Informationen versehen sind, welche keiner unabhängigen Prüfung und/oder Zertifizierung, z. B. im Hinblick auf Richtigkeit, unterzogen wurden; mithin sind bekannte Anordnungen technisch nicht derart konfiguriert, dass sie einem Verwender verlässliche Informationen zu dem in der Anordnung aufzunehmenden oder aufgenommenen Material bereitgestellt werden.

Hiervon ausgehend liegt der vorliegenden Erfindung die Aufgabe zugrunde, eine technisch verbesserte Anordnung zur Aufnahme eines erhitzbaren und/oder rauchbaren Materials, insbesondere Tabakmaterials, welches wenigstens ein Cannabinoid oder wenigstens eine wenigstens ein Cannabinoid enthaltende Verbindung enthält, anzugeben.

Die Aufgabe wird durch eine Anordnung zur Aufnahme eines wenigstens ein Cannabinoid oder wenigstens eine Cannabinoid enthaltende Verbindung enthaltenden erhitzbaren und/oder rauchbaren Materials gemäß dem unabhängigen Anspruch 1 gelöst. Die hierzu abhängigen Ansprüche betreffen mögliche Ausführungsformen der Anordnung.

Ein erster Aspekt der Erfindung betrifft eine Anordnung zur Aufnahme eines wenigstens ein Cannabinoid oder wenigstens eine wenigstens ein Cannabinoid enthaltende Verbindung enthaltenden erhitzbaren und/oder rauchbaren Materials.

Der Begriff "Cannabinoid" bezieht sich grundsätzlich auf Tetrahydrocannabinol (THC) oder Tetrahydrocannabivarin (THCV) sowie auf entsprechende Vorsubstanzen.

Mithin kann sich der Begriff "Cannabinoid" auf wenigstens eine Vorsubstanz von Tetrahydrocannabinol (THC), wie z. B. wenigstens einer durch eine chemische Reaktion, insbesondere eine Decarboxylierung, in THC umwandelbare THC-Säure, wie z. B. Tetrahydrocannabinolsäure (THCA), oder auf wenigstens eine Vorsubstanz von Tetrahydrocannabivarin (THCV) beziehen.

Im Rahmen dieses Dokuments gilt sowohl für ein Cannabinoid als auch für eine wenigstens ein Cannabinoid enthaltende Verbindung, dass diese(s) einen Gehalt an Tetrahydrocannabinol (THC) oder Tetrahydrocannabivarin (THCV) über 0,2% aufweist. Entsprechend weist ein erhitzbares und/oder rauchbares Material im Sinne des Dokuments einen Gehalt an Tetrahydrocannabinol (THC) oder Tetrahydrocannabivarin (THCV) über 0,2% auf. Analoges gilt für entsprechende Vorsubstanzen.

Insbesondere kann das erhitzbare und/oder rauchbare Material einen Gehalt an Tetrahydrocannabinol (THC) oder Tetrahydrocannabivarin (THCV) über 0,5%, über 1%, über 1,5%, über 2%, über 2,5%, über 3%, über 3,5%, über 4%, über 4,5%, über 5 %, über 5,5 %, über 6 %, über 6,5 %, über 7%, über 7,5%, über 8%, über 8,5%, über 9%, über 9,5%, über 10%, über 10,5%, über 11%, über 11,5%, über 12%, über 12,5%, über 13%, über 13,5%, über 14%, über 14,5%, über 15%, über 15,5%, über 16%, über 16,5%, über 17%, über 17,5%, über 18%, über 18,5%, über 19%, über 19,5%, über 20%, über 20,5%, über 21%, über 21,5%, über 22%, über %, über 22,5%, über 23%, über 23,5%, über 24%, über 24,5%, über 25%, über 25,5%, über 26%, über 26,5%, über 27%, über 27,5%, über 28%, über 28,5%, über 29%, über 29,5%, über 30%, über 30,5%, über 31%, über 31,5%, über 32%, über 32,5%, über 33%, über 33,5%, über 34%, über 34,5%, über 35%, über 35,5%, über 36%, über 36,5%, über 37%, über 37,5%, über 38%, über 38,5%, über 39%, über 39,5%, über 40%, über 40,5%, über41%, über 41,5%, über 42%, über 42,5%, über 43%, über 43,5%, über 44%, über 44,5%, über 45%, über 45,5%, über 46%, über 46,5%, über 47%, über 47,5%, über 48 %, über 48,5%, über 49%, über 49,5%, über 50%, über 50,5%, über 51%, über 51,5%, über 52%, über 52,5%, über 53%, über 53,5 %, über 54%, über 54,5%, über 55 55,5 %, über 56%, über 56,5%, über 57%, über 57,5%, über 58%, über 58,5%, über 59%, über 59,5%, über 60%, über 60,5%, über 61%, über 61,5%, über62%, über 62,5%, über63%, über 63,5%, über64%, über 64,5%, über 65%, über 65,5%, über 66%, über %, über 66,5 %, über 67%, über 67,5%, über 68%, über 68,5%, über 69%, über 69,5%, über 70 %, über 70,5%, über 71%, über 71,5%, über 72%, über 72,5%, über 73%, über 73,5%, über 74%, über 74,5%, über 75%, über 75,5%, über 76 %, über 76,5%, über 77%, über 77,5%, über 78%, über 78,5%, über 79%, über 79,5%, über 80%, über 80,5%, über 81 %, über 81,5%, über 82%, über 82,5%, über 83%, über 83,5%, über 84%, über 84,5%, über 85%, über 85,5%, über 86%, über 86,5%, über 87%, über 87,5%, über 88%, über 88,5%, über 89%, über 89,5%, über 90%, über 90,5%, über 91 %, über 91,5%, über 92%, über 92,5%, über 93%, über 93,5%, über 94%, über 94,5%, über 95%, über 95,5%, über 96%, über 96,5%, über 97%, über 97,5%, über 98%, über 98,5%, über 99% oder über 99,5% aufweisen. Analoges gilt für entsprechende Vorsubstanzen.

Insbesondere kann das erhitzbare und/oder rauchbare Material einen Gehalt an 100% Tetrahydrocannabinol (THC) oder Tetrahydrocannabivarin (THCV) aufweisen. Analoges gilt für entsprechende Vorsubstanzen.

Bei den vorgenannten %-Angaben handelt es sich typischerweise um Gewichtsprozent. Die vorgenannten %-Angaben können auch als Grenzwerte von Intervallen verstanden, welche einen Gehalt an THC oder THCV in dem erhitzbaren und/oder rauchbaren Produkt definieren. Das erhitzbare und/oder rauchbare Produkt kann sonach z. B. einen THC- oder THCV-Gehalt aus einem Intervall zwischen 1% und 19,5% aufweisen.

Ein entsprechendes erhitzbares und/oder rauchbares Material kann z. B. Räucherware, Rauchware, ein Raucherzeugnis, insbesondere ein rauchbares Tabakerzeugnis, sein oder einen Bestandteil wenigstens eines der Vorgenannten bilden. Insbesondere kann ein erhitzbares und/oder rauchbares Material ein erhitzbares und/oder rauchbares Tabakmaterial sein oder einen Bestandteil eines solchen bilden. Nachfolgend wird das wenigstens ein Cannabinoid oder wenigstens eine wenigstens ein Cannabinoid enthaltende Verbindung enthaltende erhitzbare und/oder rauchbare Material kurz auch als erhitzbares und/oder rauchbares Material die Rede sein. Das erhitzbare und/oder rauchbare Material kann z. B. flüssig, pastenartig bzw. -förmig oder als Feststoff vorliegen; konkret kann das erhitzbare und/oder rauchbare Material sonach z. B. granulatartig bzw.- förmig respektive partikelartig bzw. -förmig vorliegen. Auch Mischungen unterschiedlicher Aggregatzustände, z. B. Dispersionen, Suspensionen, etc. sind denkbar. Das erhitzbare und/oder rauchbare Material kann in allen Varianten oral aufnehmbar sein; der bestimmungsgemäße Konsum des erhitzbaren und/oder rauchbaren Materials kann sonach in allen Varianten oral erfolgen.

Die Anordnung stellt insgesamt eine technische Einrichtung, d. h. insbesondere ein technisches Bauteil oder eine technische Bauteilgruppe, dar, welche zur Aufnahme eines entsprechenden erhitzbaren und/oder rauchbaren Materials eingerichtet ist. Die Anordnung kann neben der Aufnahme auch zur Lagerung und/oder zum Transport eines in dieser aufgenommenen, erhitzbaren und/oder rauchbaren Materials eingerichtet sein. Die Anordnung kann dabei so beschaffen sein, dass sie eine(n) sichere(n) Aufnahme, Lagerung und/oder Transport des in dieser aufgenommenen, erhitzbaren und/oder rauchbaren Materials im Hinblick auf ein oder mehrere, sich gegebenenfalls negativ auf das in dieser aufgenommene, erhitzbare und/oder rauchbare Material auswirkenden, äußeren Einflüssen, wie z. B. chemische und/oder physikalische Einflüssen (Letztere können z. B. mechanische oder thermische Einflüsse sein), klimatische Einflüsse etc., ermöglicht.

Die Anordnung umfasst eine Aufnahmeeinrichtung, welche ein Aufnahmevolumen zur Aufnahme eines erhitzbaren und/oder rauchbaren Materials begrenzt. Die Aufnahmeeinrichtung stellt den Bestandteil der Anordnung dar, welcher der eigentlichen Aufnahme eines entsprechenden erhitzbaren und/oder rauchbaren Materials dient. Die Aufnahmeeinrichtung dient insofern typischerweise auch der Lagerung und/oder Transport von in dieser aufgenommenem erhitzbaren und/oder rauchbaren Material.

Die Aufnahmeeinrichtung ist typischerweise durch ein oder mehrere Flächen bzw. Wandungen gebildet, die das Aufnahmevolumen begrenzen. Das Aufnahmevolumen kann gegebenenfalls in mehrere Sub-Volumina aufgeteilt sein, welche voneinander durch eine oder mehrere Flächen bzw. Wandungen getrennt sind; jeweilige Sub-Volumina können sonach einzelne Aufnahmeabteile der Aufnahmeeinrichtung bilden, welche jeweils gesondert mit wenigstens einem entsprechenden erhitzbaren und/oder rauchbaren Material befüllbar oder befüllt sind.

Einzelne, mehrere oder alle Flächen bzw. Wandungen der Aufnahmeeinrichtung können z. B. aus flexiblem oder starrem Material bzw. flexiblen oder starren Materialstrukturen gebildet sein. In diesem Zusammenhang ist lediglich beispielhaft und damit nicht abschließend auf Kunststoffmaterial, wie z. B. spritzgießbare oder extrudierbare Thermoplasten, Fasermaterial, Holzmaterial, Metall oder Zellstoffmaterial, wie z. B. Karton, Papier, Pappe, etc., zu verweisen, welche zur Ausbildung der Aufnahmeeinrichtung eingesetzt bzw. verwendet werden können.

Die Aufnahmeeinrichtung kann sonach konkret z. B. als Beutel, Dose, Schachtel, Tasche oder Tüte ausgebildet sein. Die vorgenannten konkreten Ausführungsformen können, wie erwähnt, aus Kunststoffmaterial, wie z. B. spritzgießbaren oder extrudierbaren Thermoplasten, Fasermaterial, Holzmaterial, Metall oder Zellstoffmaterial, wie z. B. Karton, Papier, Pappe, etc. gebildet sein.

Die Anordnung umfasst weiterhin wenigstens einen an oder in der Aufnahmeeinrichtung angeordneten oder ausgebildeten Informationsträger, welcher wenigstens eine von einer externen Zertifizierungsstelle erzeugte und/oder zertifizierte Information enthält, die ein bzw. das in dem Aufnahmevolumen aufnehmbare(s), aufzunehmende(s) oder aufgenommene(s), wenigstens ein Cannabinoid oder wenigstens eine wenigstens ein Cannabinoid enthaltende Verbindung enthaltendes erhitzbares und/oder rauchbares Material betrifft. Die externe Zertifizierungsstelle kann z. B. eine zur Zertifizierung des erhitzbaren und/oder rauchbaren Materials autorisierte Einrichtung, insbesondere eine Behörde, ein Verein, etc., oder ein Hersteller des erhitzbaren und/oder rauchbaren Materials, oder ein Verarbeiter des erhitzbaren und/oder rauchbaren Materials sein. Die konkrete zertifizierende Zertifizierungsstelle ergibt sich typischerweise aus der zu zertifizierenden bzw. zertifizierten Information; beispielsweise kann eine medizinische Behörde die zertifizierende Zertifizierungsstelle sein, sofern es sich um die Zertifizierung von medizinischen Informationen, wie z. B. medizinischen Angaben oder Anforderungen, handelt, und eine sonstige rechtliche Behörde die zertifizierende Zertifizierungsstelle sein, sofern es sich um die Zertifizierung von gesetzlichen Informationen, wie z. B. gesetzlichen Angaben oder Anforderungen, handelt.

Die Aufnahmeeinrichtung ist über den Informationsträger mit einer von einer externen Zertifizierungsstelle erzeugten und/oder zertifizierten Information versehen, die das in dem Aufnahmevolumen aufnehmbare, aufzunehmende oder aufgenommene, wenigstens ein Cannabinoid oder wenigstens eine wenigstens ein Cannabinoid enthaltende Verbindung enthaltende erhitzbare und/oder rauchbare Material betrifft. Derart können einem Verwender, bei welchem es sich, wie eingangs, erwähnt, z. B. um eine Einrichtung in einer technischen Prozesskette, welche z. B. Herstellungs- und/oder Lieferprozesse beinhaltet, handeln kann, Sicherheit im Hinblick auf die Ausführung von in einer jeweiligen Prozesskette anfallenden technischen Prozessen verschafft. Die Sicherheit besteht insbesondere darin, dass einem Verwender zertifizierte und damit verlässliche Informationen, d. h. insbesondere technische Informationen, zu dem in der Aufnahmeeinrichtung aufzunehmenden oder aufgenommenen, erhitzbaren und/oder rauchbaren Material bereitgestellt werden, welche dieser im Rahmen der Ausführung von in einer jeweiligen Prozesskette anfallenden technischen Prozessen berücksichtigen kann oder muss. Analoges gilt wiederum für Endnutzer bzw. Konsumenten, aber auch für Personen, wie z. B. Ärzten, Apothekern, Händlern, etc., als weitere beispielhafte Gruppen von Verwendern, die Endnutzern bzw. Konsumenten entsprechende erhitzbare und/oder rauchbare Materialien zur Verfügung stellen.

Die zertifizierte Information ist damit typischerweise nicht, jedenfalls nicht ohne entsprechende Zertifizierung von einer Einrichtung, insbesondere im Sinne einer technischen und/oder wirtschaftlichen Einheit, erzeugt und/oder zertifiziert, die die Aufnahmeeinrichtung und/oder das in dieser aufzunehmende oder aufgenommene, erhitzbare und/oder rauchbare Material herstellt und/oder vertreibt, sondern von einer hierzu, insbesondere rechtlich und/oder wirtschaftlich, unabhängigen Einrichtung. Derart ist es nicht (mehr) oder nur sehr erschwert möglich, die Aufnahmeeinrichtung mit Informationen zu versehen, die das in der Aufnahmeeinrichtung aufzunehmende oder aufgenommene, erhitzbare und/oder rauchbare Material falsch beschreiben, falsch deklarieren oder auf sonstige Weise einen falschen Eindruck von dem in der Aufnahmeeinrichtung aufzunehmenden oder aufgenommenen, erhitzbaren und/oder rauchbaren Material erwecken (können).

Beispielsweise kann über eine entsprechende zertifizierte Information z. B. vermieden werden, dass die Aufnahmeeinrichtung mit falschen chemischen und/oder physikalischen Parametern des erhitzbaren und/oder rauchbaren Materials versehen wird, weil die zertifizierte Information nur zertifizierte chemische und/oder physikalische Parameter des erhitzbaren und/oder rauchbaren Materials beschreibt. Konkret kann derart z. B. verhindert werden, dass in seiner Zusammensetzung, z. B. durch Streckung, unerlaubt verändertes erhitzbares und/oder rauchbares Material falsch deklariert wird, was z. B. zu fehlerhaften Lagerungs-, Transport- und/oder Weiterverarbeitungsprozessen führen kann. Gleichermaßen kann über eine entsprechende zertifizierte Information sichergestellt werden, dass das erhitzbare und/oder rauchbare Material bestimmte rechtliche Anforderungen erfüllt, sodass Lagerungs-, Transport- und/oder Weiterverarbeitungsprozesse korrekt ausgeführt werden können.

Insgesamt liegt damit eine verbesserte Anordnung zur Aufnahme eines wenigstens ein Cannabinoid oder wenigstens eine wenigstens ein Cannabinoid enthaltende Verbindung enthaltenden erhitzbaren und/oder rauchbaren Materials, insbesondere eines erhitzbaren und/oder rauchbaren Tabakmaterials, vor.

Der Informationsträger kann z. B. gebildet sein durch oder aufweisen: ein oder mehrere alphanumerische Symbole und/oder ein oder mehrere graphische Symbole und/oder ein oder mehrere ein- oder mehrdimensionale maschinenlesbare optoelektronische Codes, insbesondere Barcodes, QR-Codes, ein oder mehrere Farbcodes, etc. Der Informationsträger und damit die wenigstens eine zertifizierte Information kann sonach durch Symbole oder Codes gebildet sein bzw. solche aufweisen, die von einem Mensch oder von einer Maschine lesbar sind. Insofern ist es möglich, die in dem Informationsträger beinhaltete wenigstens eine zertifizierte Information über entsprechende portable oder stationäre Leseeinrichtungen, wie z. B. Code-Reader, Code-Scanner, etc., automatisierbar oder automatisiert auszulesen und die ausgelesenen Informationen einer Datenverarbeitungseinrichtung, diese kann z. B. im Rahmen eines technischen Prozesses, insbesondere eines Autorisierungsprozesses, arbeiten, zuzuführen.

Ein Farbcode kann z. B. durch eine bestimmte Anordnung, diese kann regelmäßig oder unregelmäßig sein, eines oder mehrerer graphischer Elemente, wie z. B. Linien, Flächen, etc., in wenigstens einer bestimmten Farbe gebildet sein. Insbesondere kann ein Farbcode durch ein Muster, dieses kann regelmäßig oder unregelmäßig sein, eines oder mehrerer graphischer Elemente, wie z. B. Linien, Flächen, etc., in wenigstens einer bestimmten Farbgebung sein. Entsprechende graphische Elemente können in allen Varianten geometrisch definierte graphische Elemente, wie z. B. Kreise, Ovale, Polygone, oder geometrisch nicht definierte graphische Elemente, wie z. B. Freiformen sein.

Um die zertifizierte Information gegenüber unerwünschten Verwendungen und/oder Veränderungen zu schützen, kann diese verschlüsselt sein. Hierfür können gängige Verschlüsselungsprinzipien, welche gegebenenfalls auch Prinzipien der Block-Chain beinhalten oder implementieren können, eingesetzt bzw. verwendet werden.

In analoger Weise kann die zertifizierte Information wenigstens einen Link enthalten bzw. verlinkt sein; die zertifizierte Information kann sonach auch durch wenigstens einen Link bzw. Hyperlink zu einer Datenressource, wie z. B. eine Webseite in einem Netzwerk, wie z. B. dem Internet, gebildet sein bzw. wenigstens einen entsprechenden Link umfassen.

Nachfolgend werden in nicht abschließender Weise Beispiele für entsprechende zertifizierte Informationen gegeben; der Informationsträger kann einzelne, mehrere oder alle der nachfolgend gegebenen zertifizierten Informationen beinhalten.

Der Informationsträger kann eine oder mehrere zertifizierte Informationen zur Art des in der Aufnahmeeinrichtung aufzunehmenden oder aufgenommenen, erhitzbaren und/oder rauchbaren Materials beinhalten. Eine zertifizierte Information kann sonach eine oder mehrere zertifizierte Angaben zur Art des in der Aufnahmeeinrichtung aufzunehmenden oder aufgenommenen, erhitzbaren und/oder rauchbaren Materials beschreiben. Bei der Art des in der Aufnahmeeinrichtung aufzunehmenden oder aufgenommenen, erhitzbaren und/oder rauchbaren Materials kann es sich z. B. um die Gattung und die sich daraus ergebende bestimmungsgemäße Verwendung des erhitzbaren und/oder rauchbaren Materials handeln. Konkret kann eine entsprechende Angabe z. B. sein: "erhitzbares THC oder THC enthaltendes Material zur Verwendung als Räucherware", "rauchbares THC oder THC enthaltendes Material zur Verwendung als Rauchtabak", etc. In analoger Weise könnte entsprechende Angabe z. B. sein: "erhitzbares THCV oder THCV enthaltendes Material zur Verwendung als Räucherware", "rauchbares THCV oder THCV enthaltendes Material zur Verwendung als Rauchtabak", etc. sein.

Alternativ oder ergänzend kann der Informationsträger eine oder mehrere zertifizierte Informationen zur chemischen und/oder pharmazeutischen Zusammensetzung, d. h. im Allgemeinen zu chemischen und/oder pharmazeutischen Parametern, des in der Aufnahmeeinrichtung aufzunehmenden oder aufgenommenen, erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zur chemischen und/oder pharmazeutischen Zusammensetzung des in der Aufnahmeeinrichtung aufzunehmenden oder aufgenommenen, erhitzbaren und/oder rauchbaren Materials beschreiben. Entsprechende Angaben können z. B. einzelne, mehrere oder alle chemischen und/oder pharmazeutischen Bestandteile, gegebenenfalls mit Konzentration, des erhitzbaren und/oder rauchbaren Materials beinhalten.

Weiter alternativ oder ergänzend kann der Informationsträger eine oder mehrere zertifizierte Informationen zur physikalischen Zusammensetzung, d. h. im Allgemeinen zu physikalischen Parametern, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zur physikalischen Zusammensetzung des in der Aufnahmeeinrichtung aufzunehmenden oder aufgenommenen erhitzbaren und/oder rauchbaren Materials beschreiben. Entsprechende Angaben können z. B. einzelne, mehrere oder alle physikalischen Parameter, wie z. B. Brennverhalten, Gewicht, Dichte, Volumen, Feuchtigkeit, Trockenheit, Abmessungen, Form, Größe, etc., des erhitzbaren und/oder rauchbaren Materials oder dessen Bestandteile beinhalten.

Weiter alternativ oder ergänzend kann der Informationsträger eine oder mehrere zertifizierte Informationen zur Menge, insbesondere zur Masse und/oder zum Volumen, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Diese zertifizierte Information kann eine Untergruppe des bereits erwähnten physikalischen Parameters des in der Aufnahmeeinrichtung aufzunehmenden oder aufgenommenen erhitzbaren und/oder rauchbaren Materials oder wenigstens eines Bestandteils desselben sein, sodass die vorstehenden Ausführungen analog gelten.

Weiter alternativ oder ergänzend kann der Informationsträger eine oder mehrere zertifizierte Informationen zur Herkunft, insbesondere zu einem Hersteller und/oder Verarbeiter, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zur Herkunft des in der Aufnahmeeinrichtung aufzunehmenden oder aufgenommenen erhitzbaren und/oder rauchbaren Materials beschreiben. Entsprechende Angaben können z. B. einzelne, mehrere oder alle örtlichen Angaben zur Herkunft des erhitzbaren und/oder rauchbaren Materials oder eines diesem vorausgehenden Rohmaterials, Angaben zur Herkunft von Verarbeitungs- und/oder Verpackungsbetrieben, etc. des erhitzbaren und/oder rauchbaren Materials oder dessen Bestandteilen beinhalten.

Weiter alternativ oder ergänzend kann der Informationsträger eine oder mehrere zertifizierte Informationen zur Qualität, insbesondere Reinheit, und/oder einer durchgeführten Qualitätskontrolle des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zur Qualität, insbesondere Reinheit, und/oder einer durchgeführten Qualitätskontrolle des in der Aufnahmeeinrichtung aufzunehmenden oder aufgenommenen erhitzbaren und/oder rauchbaren Materials beschreiben. Entsprechende Angaben können z. B., insbesondere amtlich anerkannte, Qualitätssiegel des erhitzbaren und/oder rauchbaren Materials und/oder Qualitätssiegel von durchgeführten Qualitätskontrollen des erhitzbaren und/oder rauchbaren Materials und/oder Qualitätssigel von entsprechende Qualitätskontrollen durchführenden Qualitätskontrolleinrichtungen sein.

Weiter alternativ oder ergänzend kann der Informationsträger eine oder mehrere zertifizierte Informationen zu wenigstens einem Fertigungs- und/oder Verarbeitungs- und/oder Lagerungs- und/oder Transportprozess, insbesondere Ort und/oder Dauer und/oder Zeit des wenigstens einen Fertigungs- und/oder Verarbeitungs- und/oder Lagerungs- und/oder Transportprozesses, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zu wenigstens einem Fertigungs- und/oder Verarbeitungs- und/oder Lagerungs- und/oder Transportprozess des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beschreiben. Entsprechende Angaben können z. B. Ort- und/oder Zeitstempel jeweiliger Fertigungs- und/oder Verarbeitungs- und/oder Lagerungs- und/oder Transportprozesse sein. Derart kann eine entsprechende zertifizierte Information die Fertigungs- und/oder Verarbeitungs- und/oder Lagerungs- und/oder Transportkette des in der Aufnahmeeinrichtung aufzunehmenden oder aufgenommenen erhitzbaren und/oder rauchbaren Materials beschreiben.

Weiter alternativ oder ergänzend kann der Informationsträger eine oder mehrere zertifizierte Informationen zum bestimmungsgemäßen Konsum und/oder Konsumverhalten des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zum bestimmungsgemäßen Konsum und/oder Konsumverhalten des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beschreiben. Entsprechende Angaben können z. B. beschreiben, wie, wann, wie oft und von wem das erhitzbare und/oder rauchbare Material zu konsumieren ist. Entsprechende Angaben können gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zu einer Beschränkung, insbesondere einer Altersbeschränkung, im Zusammenhang mit dem bestimmungsgemäßen Konsum des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zu einer Altersbeschränkung im Zusammenhang mit dem bestimmungsgemäßen Konsum des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beschreiben. Entsprechende Angaben können z. B. beschreiben, ab welchem Alter und/oder bis zu welchem Alter das erhitzbare und/oder rauchbare Material konsumiert werden kann. Analoges gilt für Personenkreise, welchen der Konsum des erhitzbaren und/oder rauchbaren Materials, z. B. aus medizinischen Gründen, nicht gestattet ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zur bestimmungsgemäßen Aufbewahrung und/oder zur Haltbarkeit des in der Aufnahmeeinrichtung aufzunehmenden oder aufgenommenen erhitzbaren und/oder rauchbaren Materials beinhalten. Eine zertifizierte Information kann sonach eine oder mehrere zertifizierte Angaben zur bestimmungsgemäßen Aufbewahrung und/oder zur Haltbarkeit des in der Aufnahmeeinrichtung aufzunehmenden oder aufgenommenen erhitzbaren und/oder rauchbaren Materials beschreiben. Entsprechende Angaben können z. B. beschreiben, unter welchen Bedingungen, d. h. insbesondere chemischen und/oder physikalischen Bedingungen, das erhitzbare und/oder rauchbare Material zu lagern ist, oder Angaben zu einem minimalen oder maximalen Haltbarkeitszeitpunkt, insbesondere im Zusammenhang mit einer bestimmten Lagerung, des erhitzbaren und/oder rauchbaren Materials beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zur Rezept- und/oder Verschreibungspflichtigkeit des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zur Rezept- und/oder Verschreibungspflichtigkeit des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beschreiben. Entsprechende Angaben können z. B. beschreiben, dass das erhitzbare und/oder rauchbare Material rezept- und/oder verschreibungspflichtig ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zur Wirkung, insbesondere auch zu etwaigen Risiken und Nebenwirkungen, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials auf den menschlichen Organismus beinhalten. Eine zertifizierte Information kann sonach eine oder mehrere zertifizierte Angaben zur, insbesondere medizinischen und/oder pharmakologischen, Wirkung des erhitzbaren und/oder rauchbaren Materials auf den menschlichen Organismus beschreiben. Entsprechende Angaben können z. B. eine bestimmte, insbesondere medizinische und/oder pharmakologische, Wirkung des erhitzbaren und/oder rauchbaren Materials auf den menschlichen Organismus beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zu dem wirtschaftlichen Wert, insbesondere dem Verkaufspreis, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beinhalten. Eine zertifizierte Information kann sonach eine oder mehrere zertifizierte Angaben zu dem wirtschaftlichen Wert, insbesondere dem Verkaufspreis, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials beschreiben. Entsprechende Angaben können z. B. einen Verkaufspreis des erhitzbaren und/oder rauchbaren Materials mit oder ohne möglicherweise anfallenden Steuern beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte steuer- und/oder zollrechtliche Informationen, insbesondere von der die wenigstens eine zertifizierte Information zertifizierenden externen Zertifizierungsstelle autorisiert, beinhalten. Eine zertifizierte Information kann sonach eine oder mehrere zertifizierte steuer- und/oder zollrechtliche Angaben beschreiben. Entsprechende Angaben können z. B. angeben oder anzeigen, dass das erhitzbare und/oder rauchbare Material ordnungs- bzw. vorschriftsgemäß versteuert wurde. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger eine oder mehrere zertifizierte Informationen zu der örtlichen und/oder zeitlichen Gültigkeit der wenigstens einen zertifizierten Information beinhalten. Eine zertifizierte Information kann sonach eine oder mehrere Angaben zu ihrer örtlichen und/oder zeitlichen Gültigkeit beschreiben. Entsprechende Angaben können z. B. angeben oder anzeigen, wo und/oder wie lange die zertifizierte Information gültig ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Hinsichtlich der geometrisch-konstruktiven sowie funktionellen Gestaltung des Informationsträgers sind folgende Ausführungsformen denkbar:
Der Informationsträger kann mit dem menschlichen Auge sichtbar oder mit dem menschlichen Auge unsichtbar an oder in der Aufnahmeeinrichtung angebracht ist. Der Informationsträger kann sonach z. B. außenseitig an einer Fläche oder Wand der Aufnahmeeinrichtung angeordnet oder ausgebildet sein, sodass er mit dem menschlichen Auge sichtbar ist; ein Verwender kann sonach erkennen, dass die Aufnahmeeinrichtung mit einer zertifizierten Information versehen ist. Alternativ kann der Informationsträger in eine Fläche oder Wand der Aufnahmeeinrichtung integriert sein, sodass dieser nur durch technische Einrichtungen, wie z. B. Scanner, erkannt werden kann; derart können z. B. Manipulationsversuche des Informationsträgers verhindert oder erschwert werden.

Der Informationsträger kann unlösbar an der Aufnahmeeinrichtung angebracht sein. Insbesondere kann der Informationsträger unlösbar an der Aufnahmeeinrichtung angebracht sein, sodass er nicht beschädigungs- bzw. zerstörungsfrei von der Aufnahmeeinrichtung lösbar ist. Derart ist eine stabile Anbringung bzw. Befestigung des Informationsträgers an der Aufnahmeeinrichtung sichergestellt, welche ein unerwünschtes und gegebenenfalls unautorisiertes Entfernen des Informationsträgers von der Aufnahmeeinrichtung verhindert oder erschwert. Eine entsprechende unlösbare Anbringung kann je nach konkreter Konfiguration des Informationsträgers und der Aufnahmeeinrichtung durch form- und/oder kraft- und/oder stoffschlüssige Befestigungsprinzipien realisiert werden; lediglich beispielhaft wird in diesem Zusammenhang auf Klemmen, Spannen, Rasten, Pressen, Schrauben, Nieten, Schweißen, Löten, Kleben, etc. verwiesen.

In einer konkreten Ausführungsform kann der Informationsträger an einer freiliegenden Fläche, insbesondere einer freiliegenden Außenfläche, der Aufnahmeeinrichtung angebracht bzw. befestigt sein. Der Informationsträger kann dabei form- und/oder kraft- und/oder stoffschlüssig mit der Aufnahmeeinrichtung verbunden sein. Eine stoffschlüssige Verbindung kann auch durch Bedrucken oder Bekleben der Aufnahmeeinrichtung realisiert sein. Der Informationsträger kann sonach in fertigungs- bzw. prozesstechnisch regelmäßig einfacher und damit effizienter Weise durch Bedrucken oder Bekleben auf einer freiliegenden Fläche, insbesondere einer freiliegenden Außenfläche, der Aufnahmeeinrichtung angebracht sein.

Der Informationsträger kann eine Trägerstruktur aufweisen, welche wenigstens eine Fläche aufweist, auf welcher die zertifizierte Information angeordnet oder ausgebildet ist. Eine entsprechende Trägerstruktur kann z. B. aus Zellstoff oder einem Zellstoff enthaltenden Material, insbesondere Papier, gebildet sein. Die mit dem Informationsträger versehene Trägerstruktur kann sonach z. B. als eine Banderole oder als ein Bestandteil einer solchen ausgebildet sein. In anderen Ausführungsformen kann die Trägerstruktur aus anderen Materialien oder Materialstrukturen, wie z. B. Kunststoffmaterialien, Metallen, etc., gebildet sein; die Trägerstruktur und damit auch der Informationsträger können sonach eigenständige Bauteile bzw. Bauteilgruppen bilden, welche gesondert an der Aufnahmeeinrichtung befestigbar bzw. befestigt sind. Wiederum sei in diesem Zusammenhang auf form- und/oder kraft- und/oder stoffschlüssige Befestigungs- bzw. Verbindungsarten verwiesen, über welche eine entsprechend konfigurierte Trägerstruktur nebst Informationsträger an der Aufnahmeeinrichtung befestigbar bzw. befestigt ist.

Die Aufnahmeeinrichtung kann wenigstens eine über wenigstens ein Verschlusselement, insbesondere reversibel, verschließbare Öffnung aufweisen, über welche ein erhitzbares und/oder rauchbares Material in das Aufnahmevolumen einbringbar und/oder über welche ein erhitzbares und/oder rauchbares Material aus dem Aufnahmevolumen ausbringbar ist. Das Verschlusselement kann mit dem Informationsträger gekoppelt, d. h. insbesondere bewegungsgekoppelt, sein, sodass der Informationsträger beim Öffnen, insbesondere beim erstmaligen Öffnen der Öffnung durch einen Nutzer, in wenigstens einer Eigenschaft verändert wird. Hierunter kann gegebenenfalls auch eine Beschädigung oder Zerstörung des Informationsträgers zu verstehen sein. Der Informationsträger kann sonach mit einem oder mehreren Bereichen, d. h. beispielsweise Sollbruchbereichen, versehen sein, welche beim erstmaligen Öffnen der Aufnahmeeinrichtung, insbesondere durch entsprechendes Bewegen des Verschlusselements, beschädigt oder zerstört werden.

Um die Herstellung und/oder Handhabung der Anordnung zu vereinfachen, kann die Aufnahmeeinrichtung wenigstens eine im Hinblick auf eine Normierungsvorschrift normierte geometrisch-konstruktive Abmessung und/oder eine im Hinblick auf eine Normierungsvorschrift normierte Formgebung aufweisen. Die Aufnahmeeinrichtung kann sonach z. B. eine normierte quaderförmige oder zylindrische Geometrien aufweisen.

Ein zweiter Aspekt der Erfindung betrifft ein erhitzbares und/oder rauchbares Produkt, welches eine Anordnung nach dem ersten Aspekt der Erfindung umfasst, wobei in dem Aufnahmevolumen der Aufnahmeeinrichtung wenigstens ein erhitzbares und/oder rauchbares Material aufgenommen ist. Sämtliche Ausführungen im Zusammenhang mit der Anordnung nach dem ersten Aspekt der Erfindung gelten analog für das erhitzbare und/oder rauchbare Produkt nach dem zweiten Aspekt der Erfindung.

Ein dritter Aspekt der Erfindung betrifft ein medizinisches Produkt, welches ein erhitzbares und/oder rauchbares Produkt nach dem zweiten Aspekt der Erfindung umfasst. Sämtliche Ausführungen im Zusammenhang mit der Anordnung nach dem ersten Aspekt der Erfindung und dem erhitzbaren und/oder rauchbaren Produkt nach dem zweiten Aspekt der Erfindung gelten analog für das medizinische Produkt nach dem dritten Aspekt der Erfindung.

Ein vierter Aspekt der Erfindung betrifft ein Verfahren zur Herstellung einer Anordnung zur Aufnahme eines wenigstens ein Cannabinoid oder wenigstens eine Cannabinoid enthaltende Verbindung enthaltenden erhitzbaren und/oder rauchbaren Materials, insbesondere einer Anordnung nach dem ersten Aspekt der Erfindung. Das Verfahren umfasst insbesondere die Schritte: Bereitstellen oder Herstellen einer Aufnahmeeinrichtung, welche ein Aufnahmevolumen zur Aufnahme eines wenigstens ein Cannabinoid oder wenigstens eine wenigstens ein Cannabinoid enthaltende Verbindung enthaltenden erhitzbaren und/oder rauchbaren Materials begrenzt, und Anbringen wenigstens eines Informationsträgers, welcher wenigstens eine von einer externen Zertifizierungsstelle erzeugte und/oder zertifizierte, das in dem Aufnahmevolumen aufnehmbare bzw. aufgenommene, erhitzbare und/oder rauchbare Material betreffende zertifizierte Information enthält, an der Aufnahmeeinrichtung. Sämtliche Ausführungen im Zusammenhang mit der Anordnung nach dem ersten Aspekt der Erfindung gelten analog für das Verfahren nach dem vierten Aspekt der Erfindung.

Der zweite Schritt des Verfahrens kann insbesondere mit einer technischen Anbringungseinrichtung, wie z. B. einer Bedruckungs- und/oder Klebeeinrichtung, erfolgen. Das Verfahren kann gegebenenfalls auch einen Schritt des Erzeugens und/oder Zertifizierens einer zertifizierten Information beinhalten, welche an der Aufnahmeeinrichtung anzubringen ist bzw. an dieser angebracht wird.

Die Erfindung ist nachfolgend unter Bezugnahme auf die in den Figuren dargestellten Ausführungsbeispiele nochmals erläutert. Dabei zeigen:

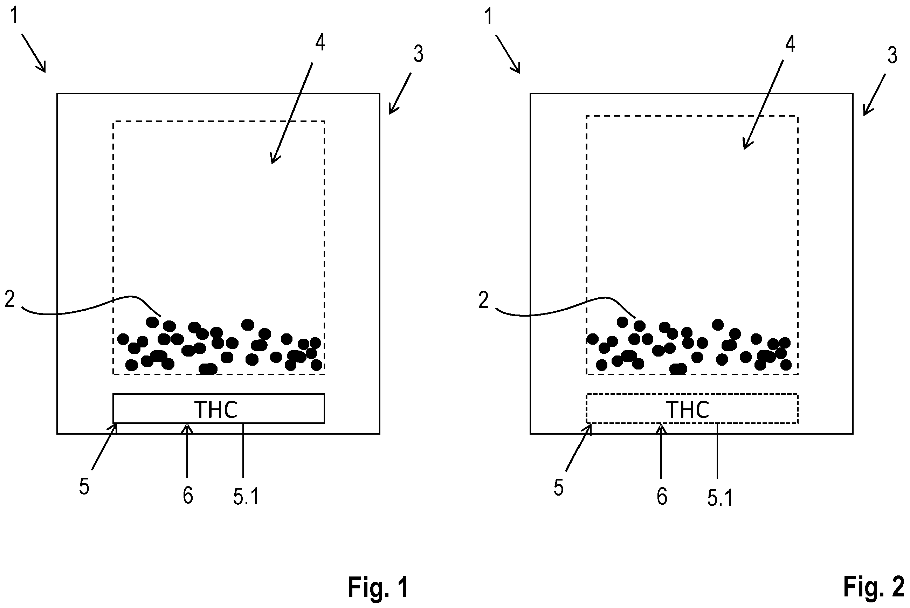

Fig. 1 bis 4 je eine schematische Prinzipdarstellung einer Anordnung gemäß einem Ausführungsbeispiel.

Fig. 1 zeigt eine Prinzipdarstellung einer Anordnung 1 zur Aufnahme eines wenigstens ein Cannabinoid oder wenigstens eine wenigstens ein Cannabinoid enthaltende Verbindung enthaltenden erhitzbaren und/oder rauchbaren Materials 2. Sowohl für ein Cannabinoid als auch für eine wenigstens ein Cannabinoid enthaltende Verbindung gilt, dass diese(s) einen Gehalt an Tetrahydrocannabinol (THC) oder Tetrahydrocannabivarin (THCV) über 0,2% aufweist. Das erhitzbare und/oder rauchbare Material 2 enthält sonach einen Gehalt an Tetrahydrocannabinol (THC) oder Tetrahydrocannabivarin (THCV) über 0,2%.

Das, wie in den Fig. angedeutet, beispielsweise granulatartig bzw. -förmige, erhitzbare und/oder rauchbare Material 2 kann z. B. Räucherware, Rauchware, ein Raucherzeugnis, insbesondere ein rauchbares Tabakerzeugnis, sein oder einen Bestandteil wenigstens eines der Vorgenannten bilden. Insbesondere kann ein erhitzbares und/oder rauchbares Material ein erhitzbares und/oder rauchbares Tabakmaterial sein oder einen Bestandteil eines solchen bilden.

Die Anordnung 1 stellt insgesamt eine technische Einrichtung, d. h. insbesondere ein technisches Bauteil oder eine technische Bauteilgruppe, dar, welche zur Aufnahme eines entsprechenden erhitzbaren und/oder rauchbaren Materials 2 eingerichtet ist. Die Anordnung 1 ist neben der Aufnahme typischerweise auch zur Lagerung und/oder zum Transport eines in dieser aufgenommenen erhitzbaren und/oder rauchbaren Materials 2 eingerichtet. Die Anordnung 1 kann dabei so beschaffen sein, dass sie eine(n) sichere(n) Aufnahme, Lagerung und/oder Transport des in dieser aufgenommenen erhitzbaren und/oder rauchbaren Materials 2 im Hinblick auf ein oder mehrere, sich gegebenenfalls negativ auf das in dieser aufgenommene, erhitzbare und/oder rauchbare Material auswirkenden, äußeren Einflüssen, wie z. B. chemische und/oder physikalische Einflüssen (Letztere können z. B. mechanische oder thermische Einflüsse sein), klimatische Einflüsse etc., ermöglicht.

Die Anordnung 1 umfasst eine Aufnahmeeinrichtung 3, welche ein Aufnahmevolumen 4 zur Aufnahme eines entsprechenden erhitzbaren und/oder rauchbaren Materials 2 begrenzt. Die Aufnahmeeinrichtung 3 stellt den Bestandteil der Anordnung 1 dar, welcher der eigentlichen Aufnahme eines entsprechenden erhitzbaren und/oder rauchbaren Materials 2 dient. Die Aufnahmeeinrichtung 3 dient insofern typischerweise auch der Lagerung und/oder Transport von in dieser aufgenommenem erhitzbaren und/oder rauchbaren Material 2.

Die Aufnahmeeinrichtung 3 ist durch ein oder mehrere Flächen bzw. Wandungen (nicht näher bezeichnet) gebildet, die das Aufnahmevolumen 4 begrenzen. Wenngleich in den Fig. nicht gezeigt, kann das Aufnahmevolumen 4 gegebenenfalls in mehrere Sub-Volumina aufgeteilt sein, welche voneinander durch eine oder mehrere Flächen bzw. Wandungen getrennt sind; jeweilige Sub-Volumina können sonach einzelne Aufnahmeabteile der Aufnahmeeinrichtung 3 bilden, welche jeweils gesondert mit wenigstens einem entsprechenden erhitzbaren und/oder rauchbaren Material 2 befüllbar oder befüllt sind.

Einzelne, mehrere oder alle Flächen bzw. Wandungen der Aufnahmeeinrichtung 3 können z. B. aus flexiblem oder starrem Material bzw. flexiblen oder starren Materialstrukturen gebildet sein. In diesem Zusammenhang ist lediglich beispielhaft und damit nicht abschließend auf Kunststoffmaterial, wie z. B. spritzgießbare oder extrudierbare Thermoplasten, Fasermaterial, Holzmaterial, Metall oder Zellstoffmaterial, wie z. B. Karton, Papier, Pappe, etc., zu verweisen, welche zur Ausbildung der Aufnahmeeinrichtung eingesetzt bzw. verwendet werden können.

Die Aufnahmeeinrichtung 3 kann sonach konkret z. B. als Beutel, Dose, Schachtel, Tasche oder Tüte ausgebildet sein. Die vorgenannten konkreten Ausführungsformen können, wie erwähnt, aus Kunststoffmaterial, wie z. B. spritzgießbaren oder extrudierbaren Thermoplasten, Fasermaterial, Holzmaterial, Metall oder Zellstoffmaterial, wie z. B. Karton, Papier, Pappe, etc. gebildet sein.

Die Anordnung 1 umfasst wenigstens einen an oder in der Aufnahmeeinrichtung 3 angeordneten oder ausgebildeten Informationsträger 5, welcher wenigstens eine von einer externen Zertifizierungsstelle erzeugte und/oder zertifizierte Information 6 enthält, die ein bzw. das in dem Aufnahmevolumen 4 aufnehmbare(s), aufzunehmende(s) oder aufgenommene(s), erhitzbares und/oder rauchbares Material 2 betrifft. Die externe Zertifizierungsstelle kann z. B. eine zur Zertifizierung des erhitzbaren und/oder rauchbaren Materials 2 autorisierte Einrichtung, insbesondere eine Behörde, ein Verein, etc., oder ein Hersteller des erhitzbaren und/oder rauchbaren Materials 2, oder ein Verarbeiter des erhitzbaren und/oder rauchbaren Materials 2 sein. Die konkrete zertifizierende Zertifizierungsstelle ergibt sich typischerweise aus der zu zertifizierenden bzw. zertifizierten Information; beispielsweise kann eine medizinische Behörde die zertifizierende Zertifizierungsstelle sein, sofern es sich um die Zertifizierung von medizinischen Informationen, wie z. B. medizinischen Angaben oder Anforderungen, handelt, und eine sonstige rechtliche Behörde die zertifizierende Zertifizierungsstelle sein, sofern es sich um die Zertifizierung von gesetzlichen Informationen, wie z. B. gesetzlichen Angaben oder Anforderungen, handelt.

Die Aufnahmeeinrichtung 3 ist sonach über den Informationsträger 5 mit einer von einer externen Zertifizierungsstelle erzeugten und/oder zertifizierten Information 6 versehen, die das in dem Aufnahmevolumen 4 aufnehmbare, aufzunehmende oder aufgenommene, erhitzbare und/oder rauchbare Material 2 betrifft. Derart können einem Verwender, bei welchem es sich, wie eingangs, erwähnt, z. B. um eine Einrichtung in einer technischen Prozesskette, welche z. B. Herstellungs- und/oder Lieferprozesse beinhaltet, handeln kann, Sicherheit im Hinblick auf die Ausführung von in einer jeweiligen Prozesskette anfallenden technischen Prozessen verschafft. Die Sicherheit besteht insbesondere darin, dass einem Verwender zertifizierte und damit verlässliche Informationen, d. h. insbesondere technische Informationen, zu dem in der Aufnahmeeinrichtung 3 aufzunehmenden oder aufgenommenen erhitzbaren und/oder rauchbaren Material 2 bereitgestellt werden, welche dieser im Rahmen der Ausführung von in einer jeweiligen Prozesskette anfallenden technischen Prozessen berücksichtigen kann oder muss. Analoges gilt wiederum für Endnutzer bzw. Konsumenten, aber auch für Personen, wie z. B. Ärzten, Apothekern, Händlern, etc., als weitere beispielhafte Gruppen von Verwendern, die Endnutzern bzw. Konsumenten entsprechende erhitzbare und/oder rauchbare Materialien 2 zur Verfügung stellen.

Die zertifizierte Information 6 ist damit typischerweise nicht, jedenfalls nicht ohne entsprechende Zertifizierung von einer Einrichtung, insbesondere im Sinne einer technischen und/oder wirtschaftlichen Einheit, erzeugt und/oder zertifiziert, die die Aufnahmeeinrichtung 3 und/oder das in dieser aufzunehmende oder aufgenommene, erhitzbare und/oder rauchbare Material 2 herstellt und/oder vertreibt, sondern von einer hierzu, insbesondere rechtlich und/oder wirtschaftlich, unabhängigen Einrichtung. Derart ist es nicht (mehr) oder nur sehr erschwert möglich, die Aufnahmeeinrichtung 3 mit Informationen zu versehen, die das in der Aufnahmeeinrichtung 3 aufzunehmende oder aufgenommene, erhitzbare und/oder rauchbare Material falsch beschreiben, falsch deklarieren oder auf sonstige Weise einen falschen Eindruck von dem in der Aufnahmeeinrichtung 3 aufzunehmenden oder aufgenommenen erhitzbaren und/oder rauchbaren Material 2 erwecken (können).

Beispielsweise kann über eine entsprechende zertifizierte Information 6 z. B. vermieden werden, dass die Aufnahmeeinrichtung 3 mit falschen chemischen und/oder physikalischen Parametern des erhitzbaren und/oder rauchbaren Materials 2 versehen wird, weil die zertifizierte Information 6 nur zertifizierte chemische und/oder physikalische Parameter des erhitzbaren und/oder rauchbaren Materials 2 beschreibt. Konkret kann derart z. B. verhindert werden, dass in seiner Zusammensetzung, z. B. durch Streckung, unerlaubt verändertes erhitzbares und/oder rauchbares Material 2 falsch deklariert wird, was z. B. zu fehlerhaften Lagerungs-, Transport- und/oder Weiterverarbeitungsprozessen führen kann. Gleichermaßen kann über eine entsprechende zertifizierte Information sichergestellt werden, dass das erhitzbare und/oder rauchbare Material 2 bestimmte rechtliche Anforderungen erfüllt, sodass Lagerungs-, Transport- und/oder Weiterverarbeitungsprozesse korrekt ausgeführt werden können.

Die zertifizierte Information 6 ist in den in den Fig. gezeigten Ausführungsbeispielen lediglich beispielhaft: "THC", könnte aber alternativ oder ergänzend auch andere Angaben beinhalten oder anders dargestellt sein, wie sich nachfolgend ergibt.

Der Informationsträger5 kann z. B. gebildet sein durch oder aufweisen: ein oder mehrere alphanumerische Symbole und/oder ein oder mehrere graphische Symbole und/oder ein oder mehrere ein- oder mehrdimensionale maschinenlesbare optoelektronische Codes, insbesondere Barcodes, QR-Codes, ein oder mehrere Farbcodes, etc. Der Informationsträger 5 und damit die zertifizierte Information 6 kann sonach durch Symbole oder Codes gebildet sein bzw. solche aufweisen, die von einem Menschen oder von einer Maschine lesbar sind. Insofern ist es möglich, die in dem Informationsträger 5 beinhaltete zertifizierte Information 6 über entsprechende portable oder stationäre Leseeinrichtungen, wie z. B. Code-Reader, Code-Scanner, etc., automatisierbar oder automatisiert auszulesen und die ausgelesenen Informationen einer Datenverarbeitungseinrichtung, diese kann z. B. im Rahmen eines technischen Prozesses, insbesondere eines Autorisierungsprozesses, arbeiten, zuzuführen.

Ein Farbcode kann z. B. durch eine bestimmte Anordnung, diese kann regelmäßig oder unregelmäßig sein, eines oder mehrerer graphischer Elemente, wie z. B. Linien, Flächen, etc., in wenigstens einer bestimmten Farbe gebildet sein. Insbesondere kann ein Farbcode durch ein Muster, dieses kann regelmäßig oder unregelmäßig sein, eines oder mehrerer graphischer Elemente, wie z. B. Linien, Flächen, etc., in wenigstens einer bestimmten Farbgebung sein. Entsprechende graphische Elemente können in allen Varianten geometrisch definierte graphische Elemente, wie z. B. Kreise, Ovale, Polygone, oder geometrisch nicht definierte graphische Elemente, wie z. B. Freiformen sein.

Um die zertifizierte Information 6 gegenüber unerwünschten Verwendungen und/oder Veränderungen zu schützen, kann diese verschlüsselt sein. Hierfür können gängige Verschlüsselungsprinzipien, welche gegebenenfalls auch Prinzipien der Block-Chain beinhalten oder implementieren können, eingesetzt bzw. verwendet werden.

In analoger Weise kann die zertifizierte Information 6 wenigstens einen Link enthalten bzw. verlinkt sein; die zertifizierte Information 6 kann sonach auch durch wenigstens einen Link bzw. Hyperlink zu einer Datenressource, wie z. B. eine Webseite in einem Netzwerk, wie z. B. dem Internet, gebildet sein bzw. wenigstens einen entsprechenden Link umfassen.

Nachfolgend werden in nicht abschließender Weise Beispiele für entsprechende zertifizierte Informationen 6 gegeben; der Informationsträger 5 kann einzelne, mehrere oder alle der nachfolgend gegebenen zertifizierten Informationen 6 beinhalten.

Der Informationsträger 5 kann eine oder mehrere zertifizierte Informationen zur Art des in der Aufnahmeeinrichtung 3 aufzunehmenden oder aufgenommenen erhitzbaren und/oder rauchbaren Materials 2 beinhalten. Eine zertifizierte Information 6 kann sonach eine oder mehrere zertifizierte Angaben zur Art des in der Aufnahmeeinrichtung 3 aufzunehmenden oder aufgenommenen erhitzbaren und/oder rauchbaren Materials 2 beschreiben. Bei der Art des in der Aufnahmeeinrichtung 3 aufzunehmenden oder aufgenommenen erhitzbaren und/oder rauchbaren Materials 2 kann es sich z. B. um die Gattung und die sich daraus ergebende bestimmungsgemäße Verwendung des erhitzbaren und/oder rauchbaren Materials 2 handeln. Konkret kann eine entsprechende Angabe z. B. sein: "erhitzbares THC oder THC enthaltendes Material 2 zur Verwendung als Räucherware", "rauchbares THC oder THC enthaltendes Material zur Verwendung als Rauchtabak", etc. In analoger Weise könne eine entsprechende Angabe z. B. sein: "erhitzbares THCV oder THCV enthaltendes Material 2 zur Verwendung als Räucherware", "rauchbares THCV oder THCV enthaltendes Material zur Verwendung als Rauchtabak", etc.

Alternativ oder ergänzend kann der Informationsträger 5 eine oder mehrere zertifizierte Informationen 6 zur chemischen und/oder pharmazeutischen Zusammensetzung, d. h. im Allgemeinen zu chemischen und/oder pharmazeutischen Parametern, des in der Aufnahmeeinrichtung 3 aufzunehmenden oder aufgenommenen erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zur chemischen und/oder pharmazeutischen Zusammensetzung des in der Aufnahmeeinrichtung 3 aufzunehmenden oder aufgenommenen, erhitzbaren und/oder rauchbaren Materials 2 beschreiben. Entsprechende Angaben können z. B. einzelne, mehrere oder alle chemischen und/oder pharmazeutischen Bestandteile, gegebenenfalls mit Konzentration, des erhitzbaren und/oder rauchbaren Materials 2 beinhalten.

Weiter alternativ oder ergänzend kann der Informationsträger 5 eine oder mehrere zertifizierte Informationen zur physikalischen Zusammensetzung, d. h. im Allgemeinen zu physikalischen Parametern, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information kann sonach eine oder mehrere zertifizierte Angaben zur physikalischen Zusammensetzung des in der Aufnahmeeinrichtung 3 aufzunehmenden oder aufgenommenen, erhitzbaren und/oder rauchbaren Materials 2 beschreiben. Entsprechende Angaben können z. B. einzelne, mehrere oder alle physikalischen Parameter, wie z. B. Brennverhalten, Gewicht, Dichte, Volumen, Feuchtigkeit, Trockenheit, Abmessungen, Form, Größe, etc., des erhitzbaren und/oder rauchbaren Materials 2 oder dessen Bestandteile beinhalten.

Weiter alternativ oder ergänzend kann der Informationsträger 5 eine oder mehrere zertifizierte Informationen 6 zur Menge, insbesondere zur Masse und/oder zum Volumen, des in der Aufnahmeeinrichtung 3 aufzunehmenden oder aufgenommenen erhitzbaren und/oder rauchbaren Materials 2 oder wenigstens eines Bestandteils des erhitzbaren und/oder rauchbaren Materials 2 beinhalten. Diese zertifizierte Information 6 kann eine Untergruppe des bereits erwähnten physikalischen Parameters des in der Aufnahmeeinrichtung 3 aufzunehmenden oder aufgenommenen, erhitzbaren und/oder rauchbaren Materials 2 oder wenigstens eines Bestandteils desselben sein, sodass die vorstehenden Ausführungen analog gelten.

Weiter alternativ oder ergänzend kann der Informationsträger 5 eine oder mehrere zertifizierte Informationen 6 zur Herkunft, insbesondere zu einem Hersteller und/oder Verarbeiter, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information kann sonach eine oder mehrere zertifizierte Angaben zur Herkunft des in der Aufnahmeeinrichtung 3 aufzunehmenden oder aufgenommenen, erhitzbaren und/oder rauchbaren Materials 2 beschreiben. Entsprechende Angaben können z. B. einzelne, mehrere oder alle örtlichen Angaben zur Herkunft des erhitzbaren und/oder rauchbaren Materials 2 oder eines diesem vorausgehenden Rohmaterials, Angaben zur Herkunft von Verarbeitungs- und/oder Verpackungsbetrieben, etc. des erhitzbaren und/oder rauchbaren Materials 2 oder dessen Bestandteilen beinhalten.

Weiter alternativ oder ergänzend kann der Informationsträger 5 eine oder mehrere zertifizierte Informationen 6 zur Qualität, insbesondere Reinheit, und/oder einer durchgeführten Qualitätskontrolle des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zur Qualität, insbesondere Reinheit, und/oder einer durchgeführten Qualitätskontrolle des in der Aufnahmeeinrichtung 3 aufzunehmenden oder aufgenommenen, erhitzbaren und/oder rauchbaren Materials beschreiben. Entsprechende Angaben können z. B., insbesondere amtlich anerkannte, Qualitätssiegel des erhitzbaren und/oder rauchbaren Materials 2 und/oder Qualitätssiegel von durchgeführten Qualitätskontrollen des erhitzbaren und/oder rauchbaren Materials 2 und/oder Qualitätssigel von entsprechende Qualitätskontrollen durchführenden Qualitätskontrolleinrichtungen sein.

Weiter alternativ oder ergänzend kann der Informationsträger 5 eine oder mehrere zertifizierte Informationen 6 zu wenigstens einem Fertigungs- und/oder Verarbeitungs- und/oder Lagerungs- und/oder Transportprozess, insbesondere Ort und/oder Dauer und/oder Zeit des wenigstens einen Fertigungs- und/oder Verarbeitungs- und/oder Lagerungs- und/oder Transportprozesses, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zu wenigstens einem Fertigungs- und/oder Verarbeitungs- und/oder Lagerungs- und/oder Transportprozess des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beschreiben. Entsprechende Angaben können z. B. Ort- und/oder Zeitstempel jeweiliger Fertigungs- und/oder Verarbeitungs- und/oder Lagerungs- und/oder Transportprozesse sein. Derart kann eine entsprechende zertifizierte Information die Fertigungs- und/oder Verarbeitungs- und/oder Lagerungs- und/oder Transportkette des in der Aufnahmeeinrichtung 3 aufzunehmenden oder aufgenommenen, erhitzbaren und/oder rauchbaren Materials 2 beschreiben.

Weiter alternativ oder ergänzend kann der Informationsträger 5 eine oder mehrere zertifizierte Informationen 6 zum bestimmungsgemäßen Konsum und/oder Konsumverhalten des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zum bestimmungsgemäßen Konsum und/oder Konsumverhalten des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beschreiben. Entsprechende Angaben können z. B. beschreiben, wie, wann, wie oft und von wem das erhitzbare und/oder rauchbare Material 2 zu konsumieren ist. Entsprechende Angaben können gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zu einer Beschränkung, insbesondere einer Altersbeschränkung, im Zusammenhang mit dem bestimmungsgemäßen Konsum des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zu einer Altersbeschränkung im Zusammenhang mit dem bestimmungsgemäßen Konsum des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beschreiben. Entsprechende Angaben können z. B. beschreiben, ab welchem Alter und/oder bis zu welchem Alter das erhitzbare und/oder rauchbare Material konsumiert werden kann. Analoges gilt für Personenkreise, welchen der Konsum des erhitzbaren und/oder rauchbaren Materials 2, z. B. aus medizinischen Gründen, nicht gestattet ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zur bestimmungsgemäßen Aufbewahrung und/oder zur Haltbarkeit des in der Aufnahmeeinrichtung 3 aufzunehmenden oder aufgenommenen, erhitzbaren und/oder rauchbaren Materials 2 beinhalten. Eine zertifizierte Information 6 kann sonach eine oder mehrere zertifizierte Angaben zur bestimmungsgemäßen Aufbewahrung und/oder zur Haltbarkeit des in der Aufnahmeeinrichtung 3 aufzunehmenden oder aufgenommenen, erhitzbaren und/oder rauchbaren Materials 2 beschreiben. Entsprechende Angaben können z. B. beschreiben, unter welchen Bedingungen, d. h. insbesondere chemischen und/oder physikalischen Bedingungen, das erhitzbare und/oder rauchbare Material 2 zu lagern ist, oder Angaben zu einem minimalen oder maximalen Haltbarkeitszeitpunkt, insbesondere im Zusammenhang mit einer bestimmten Lagerung, des erhitzbaren und/oder rauchbaren Materials 2 beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zur Rezept- und/oder Verschreibungspflichtigkeit des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zur Rezept- und/oder Verschreibungspflichtigkeit des in der Aufnahmeeinrichtung aufzunehmenden oder aufgenommenen, erhitzbaren und/oder rauchbaren Materials 2 oder wenigstens eines Bestandteils des erhitzbaren und/oder rauchbaren Materials 2 beschreiben. Entsprechende Angaben können z. B. beschreiben, dass das erhitzbare und/oder rauchbare Material 2 rezept- und/oder verschreibungspflichtig ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zur Wirkung, insbesondere auch zu etwaigen Risiken und Nebenwirkungen, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 auf den menschlichen Organismus beinhalten. Eine zertifizierte Information 6 kann sonach eine oder mehrere zertifizierte Angaben zur, insbesondere medizinischen und/oder pharmakologischen, Wirkung des erhitzbaren und/oder rauchbaren Materials 2 auf den menschlichen Organismus beschreiben. Entsprechende Angaben können z. B. eine bestimmte, insbesondere medizinische und/oder pharmakologische, Wirkung des erhitzbaren und/oder rauchbaren Materials 2 auf den menschlichen Organismus beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zu dem wirtschaftlichen Wert, insbesondere dem Verkaufspreis, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beinhalten. Eine zertifizierte Information 6 kann sonach eine oder mehrere zertifizierte Angaben zu dem wirtschaftlichen Wert, insbesondere dem Verkaufspreis, des in der Aufnahmeeinrichtung 3 aufzunehmenden oder aufgenommenen, erhitzbaren und/oder rauchbaren Materials 2 oder wenigstens eines Bestandteils des erhitzbaren und/oder rauchbaren Materials 2 beschreiben. Entsprechende Angaben können z. B. einen Verkaufspreis des erhitzbaren und/oder rauchbaren Materials 2 mit oder ohne möglicherweise anfallenden Steuern beschreiben. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte steuer- und/oder zollrechtliche Informationen 6, insbesondere von der die wenigstens eine zertifizierte Information zertifizierenden externen Zertifizierungsstelle autorisiert, beinhalten. Eine zertifizierte Information 6 kann sonach eine oder mehrere zertifizierte steuer- und/oder zollrechtliche Angaben beschreiben. Entsprechende Angaben können z. B. angeben oder anzeigen, dass das erhitzbare und/oder rauchbare Material 2 ordnungs- bzw. vorschriftsgemäß versteuert wurde. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Weiter alternativ oder ergänzend kann der Informationsträger 5 eine oder mehrere zertifizierte Informationen 6 zu der örtlichen und/oder zeitlichen Gültigkeit der jeweiligen zertifizierten Information(en) 6 beinhalten. Eine zertifizierte Information 6 kann sonach eine oder mehrere Angaben zu ihrer örtlichen und/oder zeitlichen Gültigkeit beschreiben. Entsprechende Angaben können z. B. angeben oder anzeigen, wo und/oder wie lange die zertifizierte Information 6 gültig ist. Entsprechende Angaben können ebenso gegebenenfalls auf entsprechenden Richtlinien von Behörden, medizinischen Einrichtungen, Ärzten, Apothekern, Herstellern, Verarbeitern, etc. oder gesetzlichen Regelungen basieren.

Die in den Fig. rein schematisch und zudem beispielhaft mit einer viereckigen bzw. quaderförmigen Grundform gezeigte Aufnahmeeinrichtung 3 kann konkret wie folgt konfiguriert sein:
In dem Ausführungsbeispiel gemäß Fig. 1 ist der Informationsträger 5 an einer freiliegenden Außenfläche der Aufnahmeeinrichtung 3 angeordnet oder ausgebildet; der Informationsträger 5 und die zertifizierte Information 6 ist mit dem menschlichen Auge sichtbar an der Aufnahmeeinrichtung 3 angebracht.

In dem Ausführungsbeispiel gemäß Fig. 2 ist der Informationsträger 5 in eine Fläche oder Wand der Aufnahmeeinrichtung 3 integriert, sodass dieser nur durch technische Einrichtungen, wie z. B. Scanner, erkannt werden kann; derart können z. B. Manipulationsversuche des Informationsträgers verhindert oder erschwert werden.

In allen Ausführungsbeispielen kann der Informationsträger 5 unlösbar an der Aufnahmeeinrichtung 3 angebracht sein. Insbesondere kann der Informationsträger 5 unlösbar an der Aufnahmeeinrichtung 3 angebracht sein, sodass er nicht beschädigungs- bzw. zerstörungsfrei von der Aufnahmeeinrichtung 3 lösbar ist. Derart ist eine stabile Anbringung bzw. Befestigung des Informationsträgers 5 an der Aufnahmeeinrichtung 3 sichergestellt, welche ein unerwünschtes und gegebenenfalls unautorisiertes Entfernen des Informationsträgers 5 von der Aufnahmeeinrichtung 3 verhindert oder erschwert. Eine entsprechende unlösbare Anbringung kann je nach konkreter Konfiguration des Informationsträgers 5 und der Aufnahmeeinrichtung 3 durch form- und/oder kraft- und/oder stoffschlüssige Befestigungsprinzipien realisiert werden; lediglich beispielhaft wird in diesem Zusammenhang auf Klemmen, Spannen, Rasten, Pressen, Schrauben, Nieten, Schweißen, Löten, Kleben, etc. verwiesen.

In dem Ausführungsbeispiel gemäß Fig. 1 ist der Informationsträger 5, wie erwähnt, an einer freiliegenden Fläche, insbesondere einer freiliegenden Außenfläche, der Aufnahmeeinrichtung 3 angebracht bzw. befestigt. Der Informationsträger 5 ist dabei form- und/oder kraft- und/oder stoffschlüssig mit der Aufnahmeeinrichtung 3 verbunden. Eine stoffschlüssige Verbindung kann auch durch Bedrucken oder Bekleben der Aufnahmeeinrichtung 3 realisiert sein. Der Informationsträger 5 kann sonach in fertigungs- bzw. prozesstechnisch regelmäßig einfacher und damit effizienter Weise durch Bedrucken oder Bekleben auf einer freiliegenden Fläche, insbesondere einer freiliegenden Außenfläche, der Aufnahmeeinrichtung angebracht sein.

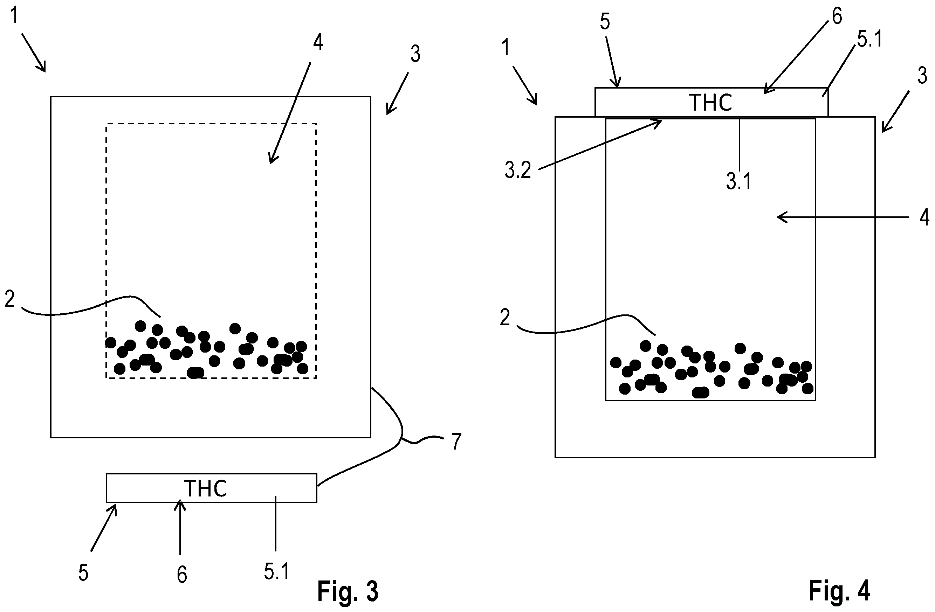

In dem Ausführungsbeispiel gemäß Fig. 3 ist der Informationsträger 5 als zu der Aufnahmeeinrichtung 3 gesondertes Bauteil ausgebildet, welches über ein Befestigungselement 7 mit der Aufnahmeeinrichtung 3 verbunden ist. Das Befestigungselement 7 kann z. B. um band-, gurt- oder kettenartig ausgebildet sein.

Der Informationsträger 5 kann in allen Ausführungsbeispielen eine Trägerstruktur 5.1 aufweisen, welche wenigstens eine Fläche aufweist, auf welcher die zertifizierte Information 6 angeordnet oder ausgebildet ist. Eine entsprechende Trägerstruktur 5.1 kann z. B. aus Zellstoff oder einem Zellstoff enthaltenden Material, insbesondere Papier, gebildet sein. Die mit dem Informationsträger 5 versehene Trägerstruktur 5.1 kann sonach z. B. als eine Banderole oder als ein Bestandteil einer solchen ausgebildet sein. Alternativ kann die Trägerstruktur 5.1 aus anderen Materialien oder Materialstrukturen, wie z. B. Kunststoffmaterialien, Metallen, etc., gebildet sein; die Trägerstruktur 5.1 und damit auch der Informationsträger 5 können sonach, wie erwähnt, eigenständige Bauteile bzw. Bauteilgruppen bilden, welche gesondert an der Aufnahmeeinrichtung 3 befestigbar bzw. befestigt sind. Wiederum sei in diesem Zusammenhang auf form- und/oder kraft- und/oder stoffschlüssige Befestigungs- bzw. Verbindungsarten verwiesen, über welche eine entsprechend konfigurierte Trägerstruktur 5.1 nebst Informationsträger an der Aufnahmeeinrichtung 3 befestigbar bzw. befestigt ist.

Anhand des in Fig. 4 gezeigten Ausführungsbeispiels ist ersichtlich, dass die Aufnahmeeinrichtung 3 wenigstens eine über wenigstens ein Verschlusselement 3.1, insbesondere reversibel, verschließbare Öffnung 3.2 aufweisen kann, über welche ein erhitzbares und/oder rauchbares Material 2 in das Aufnahmevolumen 4 einbringbar und/oder über welche ein erhitzbares und/oder rauchbares Material 2 aus dem Aufnahmevolumen 4 ausbringbar ist. Das z. B. verschwenkbar gelagerte Verschlusselement 3.1 kann mit dem Informationsträger 5 gekoppelt, d. h. insbesondere bewegungsgekoppelt, sein, sodass der Informationsträger 5 beim Öffnen, insbesondere beim erstmaligen Öffnen der Öffnung durch einen Nutzer, in wenigstens einer Eigenschaft verändert wird. Hierunter kann gegebenenfalls auch eine Beschädigung oder Zerstörung des Informationsträgers 5 zu verstehen sein. Der Informationsträger 5 kann sonach mit einem oder mehreren Bereichen, wie etwa Sollbruchbereichen, versehen sein, welche beim erstmaligen Öffnen der Aufnahmeeinrichtung 3 durch entsprechendes Bewegen des Verschlusselements 3.1 beschädigt oder zerstört werden.

Um die Herstellung und/oder Handhabung der Anordnung 1 zu vereinfachen, kann die Aufnahmeeinrichtung 3 wenigstens eine im Hinblick auf eine Normierungsvorschrift normierte geometrisch-konstruktive Abmessung und/oder eine im Hinblick auf eine Normierungsvorschrift normierte Formgebung aufweisen. Die Aufnahmeeinrichtung 3 kann sonach z. B. eine normierte quaderförmige oder zylindrische Geometrien aufweisen.

Ein erhitzbares und/oder rauchbares Produkt kann bereitgestellt werden, indem eine entsprechende Anordnung 1 in dem Aufnahmevolumen 4 der Aufnahmeeinrichtung 3 wenigstens ein erhitzbares und/oder rauchbares Material 2 aufweist.

Ein medizinisches Produkt kann bereitgestellt werden, welches ein entsprechendes erhitzbares und/oder rauchbares Produkt aufweist.

Ein Verfahren zur Herstellung einer entsprechenden Anordnung 1 zur Aufnahme eines erhitzbaren und/oder rauchbaren Materials 2 kann die folgenden Schritte umfassen: Bereitstellen oder Herstellen einer Aufnahmeeinrichtung 3, welche ein Aufnahmevolumen 4 zur Aufnahme eines erhitzbaren und/oder rauchbaren Materials 2 begrenzt, und Anbringen wenigstens eines Informationsträgers 5, welcher wenigstens eine von einer externen Zertifizierungsstelle erzeugte und/oder zertifizierte, das in dem Aufnahmevolumen 4 aufnehmbare bzw. aufgenommene, erhitzbare und/oder rauchbare Material 2 betreffende zertifizierte Information 6 enthält, an der Aufnahmeeinrichtung 3.

Der zweite Schritt des Verfahrens kann insbesondere mit einer technischen Anbringungseinrichtung, wie z. B. einer Bedruckungs- und/oder Klebeeinrichtung, erfolgen. Das Verfahren kann gegebenenfalls auch einen Schritt des Erzeugens und/oder Zertifizierens einer zertifizierten Information 6 beinhalten, welche an der Aufnahmeeinrichtung 3 anzubringen ist bzw. an dieser angebracht wird.

## Patentansprüche

1. Anordnung zur Aufnahme eines erhitzbaren und/oder rauchbaren Materials, insbesondere Tabakmaterials, welches wenigstens ein Cannabinoid oder wenigstens eine wenigstens ein Cannabinoid enthaltende Verbindung enthält, umfassend:
- eine Aufnahmeeinrichtung, welche ein Aufnahmevolumen zur Aufnahme eines wenigstens ein Cannabinoid oder wenigstens eine wenigstens ein Cannabinoid enthaltende Verbindung enthaltenden erhitzbaren und/oder rauchbaren Materials begrenzt,
- wenigstens einen an oder in der Aufnahmeeinrichtung angeordneten oder ausgebildeten Informationsträger, welcher wenigstens eine von einer externen Zertifizierungsstelle erzeugte und/oder zertifizierte Information enthält, die ein in dem Aufnahmevolumen aufnehmbares oder aufgenommenes, wenigstens ein Cannabinoid oder wenigstens eine wenigstens ein Cannabinoid enthaltende Verbindung enthaltendes erhitzbares und/oder rauchbares Material betrifft.

2. Anordnung nach Anspruch 1, wobei der Informationsträger gebildet ist durch oder aufweist: ein oder mehrere alphanumerische Symbole, und/oder ein oder mehrere graphische Symbole, und/oder ein oder mehrere ein- oder mehrdimensionale maschinenlesbare optoelektronische Codes, insbesondere Barcodes, QR-Codes, wobei die zertifizierte Information vorzugsweise verschlüsselt ist.

3. Anordnung nach einem der vorhergehenden Ansprüche, wobei der Informationsträger eine oder mehrere der folgenden zertifizierten Informationen enthält:
- eine oder mehrere zertifizierte Informationen zur Art des in der Aufnahmeeinrichtung aufzunehmenden oder aufgenommenen erhitzbaren und/oder rauchbaren Materials;
- eine oder mehrere zertifizierte Informationen zur chemischen und/oder pharmazeutischen Zusammensetzung des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur physikalischen Zusammensetzung des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur Herkunft, insbesondere zu einem Hersteller und/oder Verarbeiter, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur Qualität, insbesondere Reinheit, und/oder einer durchgeführten Qualitätskontrolle des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zu wenigstens einem Fertigungs- und/oder Verarbeitungs- und/oder Lagerungsprozess, insbesondere Ort und/oder Zeit des wenigstens einen Fertigungs- und/oder Verarbeitungs- und/oder Lagerungsprozesses, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur Menge, insbesondere zur Masse, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zum bestimmungsgemäßen Konsum und/oder Konsumverhalten des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zu einer Altersbeschränkung im Zusammenhang mit dem bestimmungsgemäßen Konsum des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur bestimmungsgemäßen Aufbewahrung und/oder zur Haltbarkeit des in der Aufnahmeeinrichtung aufzunehmenden oder aufgenommenen wen erhitzbaren und/oder rauchbaren Materials oder wenigstens eines Bestandteils des erhitzbaren und/oder rauchbaren Materials;
- eine oder mehrere zertifizierte Informationen zur Rezept- und/oder Verschreibungspflichtigkeit des in der Aufnahmeeinrichtung aufzunehmenden oder aufgenommenen erhitzbaren und/oder rauchbaren Materials oder wenigstens eines Bestandteils des erhitzbaren und/oder rauchbaren Materials;
- eine oder mehrere zertifizierte Informationen zur Wirkung, insbesondere auch zu etwaigen Risiken und Nebenwirkungen, des in der Aufnahmeeinrichtung aufzunehmenden oder aufgenommenen erhitzbaren und/oder rauchbaren Materials oder wenigstens eines Bestandteils des erhitzbaren und/oder rauchbaren Materials auf den menschlichen Organismus;
- eine oder mehrere zertifizierte Informationen zu dem wirtschaftlichen Wert, insbesondere dem Verkaufspreis, des in der Aufnahmeeinrichtung aufzunehmenden oder aufgenommenen enthaltenden erhitzbaren und/oder rauchbaren Materials oder wenigstens eines Bestandteils des erhitzbaren und/oder rauchbaren Materials;
- eine oder mehrere zertifizierte steuer- und/oder zollrechtliche Informationen, insbesondere von der die wenigstens eine zertifizierte Information zertifizierenden externen Zertifizierungsstelle autorisiert;
- eine oder mehrere zertifizierte Informationen zu der örtlichen und/oder zeitlichen Gültigkeit der wenigstens einen zertifizierten Information.

4. Anordnung nach einem der vorhergehenden Ansprüche, wobei der Informationsträger mit dem menschlichen Auge sichtbar oder mit dem menschlichen Auge unsichtbar an oder in der Aufnahmeeinrichtung angebracht ist.

5. Anordnung nach einem der vorhergehenden Ansprüche, wobei der Informationsträger unlösbar an der Aufnahmeeinrichtung angebracht ist und/oder form- und/oder kraft- und/oder stoffschlüssig mit der Aufnahmeeinrichtung verbunden ist.

6. Anordnung nach einem der vorhergehenden Ansprüche, wobei der Informationsträger an einer freiliegenden Außenfläche der Aufnahmeeinrichtung angebracht ist.

7. Anordnung nach einem der vorhergehenden Ansprüche, wobei der Informationsträger eine Trägerstruktur aufweist, welche wenigstens eine Fläche aufweist, auf welcher die zertifizierte Information angeordnet oder ausgebildet ist, wobei die Trägerstruktur insbesondere aus Zellstoff oder einem Zellstoff enthaltenden Material, insbesondere Papier, gebildet ist.

8. Anordnung nach einem der vorhergehenden Ansprüche, wobei die externe Zertifizierungsstelle eine zur Zertifizierung des erhitzbaren und/oder rauchbaren Materials autorisierte Einrichtung, insbesondere eine Behörde, ein Verein, etc., oder ein Hersteller des erhitzbaren und/oder rauchbaren Materials, oder ein Verarbeiter des erhitzbaren und/oder rauchbaren Materials ist.

9. Anordnung nach einem der vorhergehenden Ansprüche, wobei die Aufnahmeeinrichtung eine über ein Verschlusselement, insbesondere reversibel, verschließbare Öffnung aufweist, über welche ein entsprechendes erhitzbares und/oder rauchbares Material aus dem Aufnahmevolumen ausbringbar ist.

10. Anordnung nach Anspruch 9, wobei das Verschlusselement mit dem Informationsträger gekoppelt ist, sodass der Informationsträger beim Öffnen, insbesondere beim erstmaligen Öffnen der Öffnung durch einen Nutzer, in wenigstens einer Eigenschaft verändert wird.

11. Anordnung nach einem der vorhergehenden Ansprüche, wobei die Aufnahmeeinrichtung als Beutel, Dose, Schachtel, Tasche oder Tüte ausgebildet ist.

12. Anordnung nach einem der vorhergehenden Ansprüche, wobei die Aufnahmeeinrichtung wenigstens eine im Hinblick auf eine Normierungsvorschrift normierte geometrisch-konstruktive Abmessung und/oder eine im Hinblick auf eine Normierungsvorschrift normierte Formgebung aufweist.

13. Erhitzbares und/oder rauchbares Produkt, umfassend eine Anordnung nach einem der vorhergehenden Ansprüche, wobei in dem Aufnahmevolumen der Aufnahmeeinrichtung ein wenigstens ein Cannabinoid oder wenigstens eine wenigstens ein Cannabinoid enthaltende Verbindung enthaltendes erhitzbares und/oder rauchbares Material aufgenommen ist.

14. Medizinisches Produkt, umfassend ein erhitzbares und/oder rauchbares Produkt nach Anspruch 13.

15. Verfahren zur Herstellung einer Anordnung zur Aufnahme eines wenigstens ein Cannabinoid oder wenigstens eine Cannabinoid enthaltende Verbindung enthaltenden erhitzbaren und/oder rauchbaren Materials, insbesondere einer Anordnung nach einem der vorhergehenden Ansprüche, umfassend die Schritte:
- Bereitstellen oder Herstellen einer Aufnahmeeinrichtung, welche ein Aufnahmevolumen zur Aufnahme eines wenigstens ein Cannabinoid oder wenigstens eine Cannabinoid enthaltende Verbindung enthaltenden erhitzbaren und/oder rauchbaren Materials begrenzt,
- Anbringen wenigstens eines Informationsträgers, welcher wenigstens eine von einer externen Zertifizierungsstelle zertifizierte, in dem Aufnahmevolumen aufnehmbare bzw. aufgenommene wenigstens ein Cannabinoid oder wenigstens eine Cannabinoid enthaltende Verbindung enthaltende erhitzbare und/oder rauchbare Material betreffende Information enthält, an der Aufnahmeeinrichtung.
